# EUROPÄISCHE PATENTANMELDUNG

(11) **EP 1 767 234 A1**
(43) Veröffentlichungstag der Anmeldung: **28.03.2007**
(21) Anmeldenummer: 06020193.6
(22) Anmeldetag: 27.09.2006
(51) Int. Cl.: A61M 5/168

(54) **Dosiervorrichtung mit einer Tropfkammer und einer Lufteinlassöffnung**

(30) Priorität: 27.09.2005 DE 102005046101; 11.10.2005 DE 102005049024
(71) Anmelder: Human Nutrition GmbH, 55234 Bechtolsheim (DE)
(72) Erfinder: Wendel, Hermann J., 55288 Armsheim (DE)
(74) Vertreter: Wagner, Jutta

(57) **Zusammenfassung**

Dosiervorrichtung für die enterale Emährung, umfassend ein Rohr, dessen einer Teil in einen Vorratsbehälter mit einer Suspension für die enterale Emährung hineinragt und dessen anderer Teil über ein Ventil, vorzugsweise ein Nadelventil, mit einer Lufteinlassöffnung verbunden ist sowie eine Anschlussvorrichtung zur Verbindung des Vorratsbehälters mit dem Patienten, die einen Anschluss zum Verbinden mit dem Vorratsbehälter, eine Tropfkammer und eine Zuführungsleitung zum Patienten umfasst.

## Beschreibung

Die vorliegende Erfindung betrifft eine Dosiervorrichtung für Flüssigkeiten, insbesondere für Suspensionen für die enterale Ernährung.

Für die intravenöse Ernährung werden üblicherweise entsprechende, in flexiblen Plastikbeuteln eingeschlossene klare Lösungen über einen Ablass- und Verbindungsschlauch zum Patienten, welcher eine Tropfkammer und eine zur Regulierung der Ablaufgeschwindigkeit um den Ablassschlauch angeordnete Quetschvorrichtung enthält, durch die Schwerkraft gefördert. Die Quetschvorrichtung erlaubt dabei eine sehr feine Dosierung der Lösung. Da der flexible Plastikbehälter in sich zusammenfällt, braucht die entnommene Lösung nicht durch Luft ersetzt zu werden, so dass die in dem Beutel befindliche Lösung steril bleibt.

Es ist in DE 1915204 für formstabile Vorratsbehälter auch schon vorgeschlagen worden, die verbrauchte Lösung durch Luft zu ersetzen und hierbei den Luftzustrom durch ein Rückschlagventil zu begrenzen. Damit soll verhindert werden, dass die Tropfgeschwindigkeit durch die bei einem ansonsten auftretenden stoßweisen Lufteintritt verursachten Druckschwankungen beeinflusst wird, siehe DE 1915204. Die Dosierung erfolgt aber auch nach diesem Dokument mittels Quetschvorrichtung.

Im Gegensatz zu der venösen Ernährung, wobei klare Lösungen eingesetzt werden, wird zur enteralen Ernährung normalerweise eine auch feste Bestandteile enthaltende Suspension verwendet, die sich nicht in gleicher Weise applizieren lässt, da die Festbestandteile das Quetschventil nach kurzer Zeit zusetzen und blockieren würden. Es hat sich deshalb durchgesetzt, Suspensionen für die enterale Ernährung in festen, aus Glas oder Kunststoff, beispielsweise Polyethylen oder Polyurethan, bestehenden Flaschen als Lösung oder als Pulver, welches kurz vor der Applikation mit Wasser rekonstruiert wird, einzusetzen und die Lösung über ein entsprechendes Pumpsystem zu entnehmen, wobei die Pumpleistung die Applikationsgeschwindigkeit bestimmt. Das Pumpsystem macht diese Vorrichtungen vergleichsweise unhandlich und teuer.

Bis zur Anwendung sind entsprechende Flaschen üblicherweise durch einen Kronenkorken oder ähnlichen Verschluss verschlossen, welcher für die Anwendung durch einen Aufsatz ersetzt wird, der ein mit der Pumpe verbundenes Ableitungsrohr aufweist, und ein Rückschlagventil hat, durch welches Luft in den Behälter nachströmen aber die im Behälter befindliche Lösung nicht nach außen austreten kann. Im Gegensatz zur intravenösen Ernährung, bei der auf besondere Sterilität geachtet werden muss, spielt eine geringe Kontamination der enteralen Suspensionen während der Anwendung durch die einströmende Luft keine Rolle, da diese bei einer Applikation über den Magen vertragen werden. Selbstverständlich muss bis zur Applikation ein solcher Behälter steril verschlossen sein, um ein Verderben der Inhaltsstoffe zu vermeiden (vgl. US-P 4,754,891).

Aus DE 102 53 258 ist eine Vorrichtung zur Regulierung der enteralen Ernährung bekannt geworden, die aus einer formstabilen oder wenig deformierbaren Vorratsflasche besteht, welche die für die Ernährung dienende Suspension sowie einen gewissen Luftraum aufweist und über eine Verschlussvorrichtung mit der Zuführungsleitung zum Patienten verbunden ist, wobei die Verschlussvorrichtung ein rechtwinkliges Rohr enthält, dessen einer Teil in die Zuführung der Flasche hineinragt und dessen anderer Teil nach außen aus der Verschlussvorrichtung herausragt und ein Lufteinlassloch aufweist, welches durch eine Lufteinlassschraube ganz oder teilweise verschlossen werden kann, sowie eine Tropfkammer als Übergang zu der Ableitung aufweist.

Obwohl durch diese Vorrichtung eine erhebliche Vereinfachung erreicht ist, ist die Regulierung im Vergleich zu den Pumpensystemen nicht genauso präzise bzw. reproduzierbar möglich. Zudem ist das dort beschrieben System als Wegwerf-Produkt nur für den einmaligen Gebrauch vorgesehen, was eine aufwändigere Gestaltung ausschließt.

Aufgabe der vorliegenden Erfindung war es daher, eine Dosiervorrichtung für die enterale Ernährung anzugeben, die einerseits einfach und kostengünstig ist und andererseits eine präzise und reproduzierbare Regulierung erlaubt.

Diese Aufgabe wird gelöst durch eine Dosiervorrichtung, insbesondere für die enterale Ernährung, umfassend ein Rohr, dessen einer Teil in einen Vorratsbehälter mit einer Suspension für die enterale Ernährung hineinragt und dessen anderer Teil über ein präzise und über eine Skalierung reproduzierbar einstellbares Dosierventil mit einer Lufteinlassöffnung verbunden ist sowie eine Anschlussvorrichtung zur Verbindung des Vorratsbehälters mit dem Patienten, die einen Anschluss zum Verbinden mit dem Vorratsbehälter, eine Tropfkammer und eine Zuführungsleitung zum Patienten umfasst.

Die erfindungsgemäße Dosiervorrichtung erlaubt eine einfache Förderung der Suspension aus dem Vorratsbehälter über die Schwerkraft in Verbindung mit einer exakten Dosierbarkeit mittels der Regulierung über die durch ein Ventil nachströmende Luft. Somit wird die im Vergleich zu Pumpensystemen wesentlich einfachere Förderung über die Schwerkraft mittels Lufteinlass mit der präzisen und reproduzierbaren Dosierung durch ein Ventil kombiniert.

Die erfindungsgemäße Dosiervorrichtung eignet sich für alle bekannten Vorratsbehälter, welche formstabil oder nur wenig deformierbar sind, insbesondere für Glas- und Kunststoffflaschen. Es lassen sich Lösungen und Suspensionen für die enterale Ernährung dosieren. Der Einfachheit halber wird im folgenden nur von Suspension gesprochen, wobei damit auch andere Lösungen umfasst sein sollen.

Das Rohr gewährleistet die dosierte Zufuhr von Luft in den Vorratsbehälter. Es besteht aus einem nicht verformbaren Material, wie z.B. Glas, oder aus einem nur wenig verformbaren Material, wie z.B. einem steifen Kunststoffschlauch. Wichtig ist, dass das Rohr eine ausreichende Steifigkeit besitzt, die ein Zusammenfallen verhindert.

Das präzise und reproduzierbar regulierbare Ventil ist vorzugsweise ein Nadelventil, mit dem eine sehr feine Dosierung von Luft möglich ist. Es sind jedoch auch andere Arten von Dosierventilen möglich, wichtig ist allein, dass der Druchfluss exakt und mittels einer entsprechenden Skalierung auch reproduzierbar eingestellt werden kann. Dies ist mit der Lufteinlassschraube nach DE 902 53 258 so nicht möglich.

In einer ersten bevorzugten Ausführungsform handelt es sich um ein elektronisch gesteuertes Ventil. Hierbei ist auch eine programmierbare Steuerung denkbar, welche die enterale Ernährung z.B, abschnittsweise zu vorbestimmten Zeiten an- und abschalten kann. Die Skalierung wird hier vorzugsweise durch eine Umsetzung in eine elektronsiche Anzeige verwirklicht. Es ist ebenfalls möglich, einen entsprechenden z.B. Drehschalter zur Einstellung zu verwenden, dessen Stellung mit einer Skalierung die reproduzierbare Dosierung ermöglicht.

In einer anderen besonders bevorzugten Ausführungsform handelt es sich um eine handbetätigtes Nadelventil mit einer Skalierung.

Zur weiteren Verbesserung der Dosierung kann das Nadelventil mit einem Getriebe versehen werden, das den Regelbereich spreizt.

Gewünschtenfalls kann die Lufteinlassöffnung mit einem Filter oder einem Vorrat an Reinluft (oder einem anderen physiologisch verträglichen Gas) verbunden sein, um jede auch nur geringste Kontamination der Suspension zur enteralen Ernährung auszuschließen. Im folgenden wird die Erfindung anhand einer Luftzufuhr bzw. Luft erläutert, die Ausführungen gelten jedoch bei Verwendung von Reinluft bzw. einem physiologisch verträglichen Gas entsprechend.

Vorzugsweise ist ein dauerhaft bakteriendichter Filter an der Lufteinlassöffnung vorgesehen.

Sofern das Rohr an seinem in den Vorratsbehälter hineinragenden Ende sich zumindest zeitweise unterhalb der Flüssigkeitsobeffläche der Suspension befindet, wird dieses Ende mit einem Rückschlagventil gegen ein Eindringen der Suspension gesichert.

In einer besonders bevorzugten Ausführungsform ist das Rohr mit der Anschlussvorrichtung verbindbar. In die Anschlussvorrichtung ist hierbei vorzugsweise der Teil des Rohres, der in den Vorratsbehälter hineinragt, integriert. Dieser Teil des Rohres ragt durch das Anschlussteil hindurch. Es ist günstig, wenn das Rohr hierzu rechtwinklig ausgebildet ist. Die Verbindung des Rohres mit der Anschlussvorrichtung ist vorzugsweise mit einem Ventil verschließbar, insbesondere mit einem Einwegventil, wodurch ein Eindringen von Luft und damit Keimen verhindert wird. Insbesondere in Zusammenwirkung eines Ventils mit einem bakteriendichten Filter an der Lufteinlassöffnung kann somit eine weitgehende Sterilität des Dosiersystems gewährleistet werden.

Durch diese zweiteilige Ausführungsform ist auch gewährleistet, dass die preiswerten Teile des Systems, wie Anschlussvorrichtung und hineinragendes Rohr als Einweg-Systeme ausgebildet werden können, während das Ventil und andere kostenintensivere Teile wiederverwendbar sind.

Die Ausbildung der Verbindung zwischen Anschlusssystem und Rohr ist nicht weiter beschränkt. Es eignen sich z.B. rechteckige oder runde Stutzen, über die ein entsprechend ausgebildeter Teil des Rohres geschoben werden kann. Besonders einfach und sicher sind an sich bekannte Dreh- oder Schnappverschlüsse.

In einer besonders bevorzugten Ausführungsform wird ein übliches Überleitsystem für die enterale Ernährung mit einem am Ende rechteckig ausgebildeten Lufteinlass versehen. Das rechteckig ausgebildete Ende der Anschlussvorrichtung wird mit dem Rohr des erfindungsgemäßen Dosierteiles verbunden. Durch die rechteckige Ausformung wird ein Verdrehen und damit Herausrutschen aus dem Rohr verhindert.

In einer anderen Ausführungsform, die sich besonders für Vorratsbehälter aus Kunststoff eignet, sind Anschlussteil und Rohr räumlich voneinander getrennt. Das Rohr wird hierbei mit dem Ende, welches in den Vorratsbehälter ragt, an dessen oberem Ende eingeführt. Dazu sollte das Rohrende zweckmäßigerweise mit einer geeigneten Schneidkante ausgebildet sein, damit es die Öffnung in dem Vorratsbehälter durch Einstechen selber erzeugen kann. Alternativ oder zusätzlich kann der Behälter so angepasst sein, dass die Öffnung an einer geschwächten Stelle der Wandung leicht ausbildbar ist. Eine dritte Möglichkeit besteht darin, dass ein entsprechender Anschluss vorgesehen ist. Auch hierbei ergibt sich eine Mehrfachverwendung des Dosierteiles.

Die Zufuhr der dosierten Nahrung zum Patienten wird in an sich bekannter Weise durch eine in die Anschlussvorrichtung integrierte Tropfkammer mit einer daran angeschlossenen Zufuhrleitung bewirkt. Je nach Ausführungsform des Rohres mit Lufteinlass, kann es sich um eine herkömmliche Tropfkammer handeln, oder aber der in den Vorratsbehälter ragende Teil des Rohres ist integriert. Es versteht sich, dass die Tropfkammer in ihrer Dimensionierung an die jeweilige Nahrung angepasst wird.

Es ist außerdem bevorzugt, wenn in der Tropfkammer oder in der Zufuhrleitung ein Einwegeventil vorgesehen ist, welches ein Zurückfließen der Nahrung verhindert. Das Ventil kann z.B. als Lippenventil, Membranventil oder in jeder anderen bekannten Form ausgebildet sein. Vorzugsweise kommt ein Membranventil zum Einsatz. Ohne das Einwegventil ist es möglich, dass bei Bewegung, Husten etc. die Nahrung durch einen im Behälter bestehenden Unterdruck zurückgezogen wird.

Weiterhin kann das Rohr zusätzlich mit einem Absperrventil versehen sein. Dieses ermöglicht eine schnelle komplette Absperrung der Luftzufuhr und übernimmt somit die Funktion eines An- und Abschalters.

Es ist bevorzugt, den Teil des Rohres, in welchem das Ventil und die Lufteinlassöffnung angeordnet sind, in einem Gehäuse unterzubringen. Hierbei ist vorzugsweise dafür zu sorgen, dass die Betätigung des Ventils von außen erfolgen kann, entweder indem ein Griff eines handbetätigten Ventils aus dem Gehäuse nach außen ragt oder indem entsprechende Betätigungselemente eines elektronischen Ventils außen an dem Gehäuse angeordnet sind. Bei einem handbetätigten Ventil ist auch die Skalierung außen auf dem Gehäuse angeordnet. Die Unterbringung in einem Gehäuse macht die Vorrichtung leichter handhabbar, optisch ansprechender und dient auch als Schutz der Komponenten vor Verschmutzung und Beschädigung.

Mit der erfindungsgemäßen Vorrichtung lassen sich auch Flüssigkeiten für andere Zwecke auf einfache Weise mittels der Schwerkraft dosieren. Es kann sich bei den Flüssigkeiten z.B. um Lösungen, Suspensionen oder Emulsionen handeln.

Mit Bezug auf die beigefügten Figuren, in welcher eine besonders bevorzugte Ausführungsform dargestellt ist, soll die Erfindung näher erläutert werden, ohne jedoch darauf beschränkt zu sein.

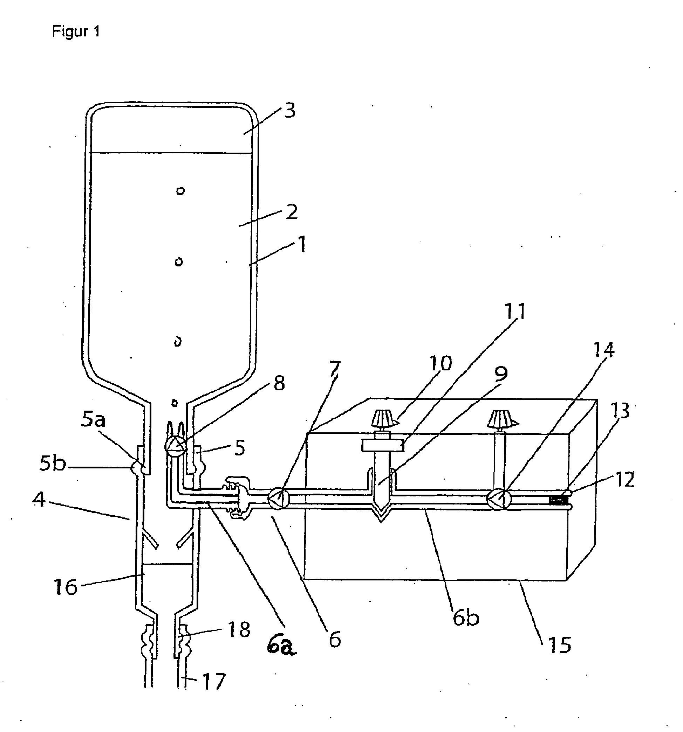

In Figur 1 ist mit 1 der Vorratsbehälter aus einem formstabilen Material mit der darin enthaltenen Suspension 2 zur enteralen Ernährung dargestellt. In dem Behälter 1 befindet sich ein Luftraum 3. Das Anschlussteil 4 ist mit einer Schnappverbindung 5, bestehend aus einem Wulst 5a an dem Behälter 1 und einer daran angepassten Nut 5b an dem Anschlussteil 4 mit dem Behälter 1 verbunden.

Das Rohr 6 ist durch ein Einwegventil 7 in einen in den Behälter ragenden Teil 6a und einen Teil 6b unterteilt. Der Teil 6a ist rechtwinklig ausgebildet. Am Ende des Teils 6a, welches in den Behälter 1 ragt, ist ein Rückschlagventil 8 angebracht, welches ein Eindringen der Suspension 2 in das Rohr 6 verhindert. Am anderen Ende des Teils 6a ist eine Verbindung zum Verbinden mit dem Teil 6b an dem Einwegventil 7 vorgesehen. Das Einwegventil 7 verhindert ein Rückströmen von Luft aus dem Teil 6a in den Teil 6b, bzw. ein Eindringen von Luft in den Teil 6b, wenn dieser nicht mit dem Teil 6a verbunden ist.

Der Teil 6b wird von einem flexiblen Kunststoffschlauch gebildet, dessen Durchmesser deutlich geringer als derjenige des Teils 6a sein kann. In dem Teil 6b sind das Nadelventil 9 mit einer Skalierung 10 und einem Getriebe 11 zur Spreizung des Regelbereiches angeordnet. Das Rohr ist am Ende des Teils 6b mit einer Lufteinlassöffnung 12 versehen, die mit einem bakteriendichten Filter 13 vor einem Eindringen von Keimen geschützt ist. Ein Absperrventil 14, mit dem das Rohr 6 schlagartig verschlossen werden kann, dient als An- und Abschaltschalter. Der Teil 6b des Rohres 6 ist größtenteils in einem Gehäuse 15 untergebracht, wobei sich die Skalierung 10 und der Teil des Nadelventils 9, mit welchem dieses betätigt wird, außerhalb des Gehäuses 15 befinden.

Das Anschlussteil 4 umfasst neben dem Rohrteil 6a eine Tropfkammer 16 und die Zuführungsleitung 17 zum Patienten. Die Verbindung zwischen Tropfkammer 16 und Zuführungsleitung 17 wird durch eine Schlaucholive 18, auf weiche die aus einem flexiblen Schlauch bestehende Zuführungsleitung 17 aufgeschoben ist, gebildet. Selbstverständlich kann die Zufuhrleitung auch fest mit der Tropfkammer verbunden sein.

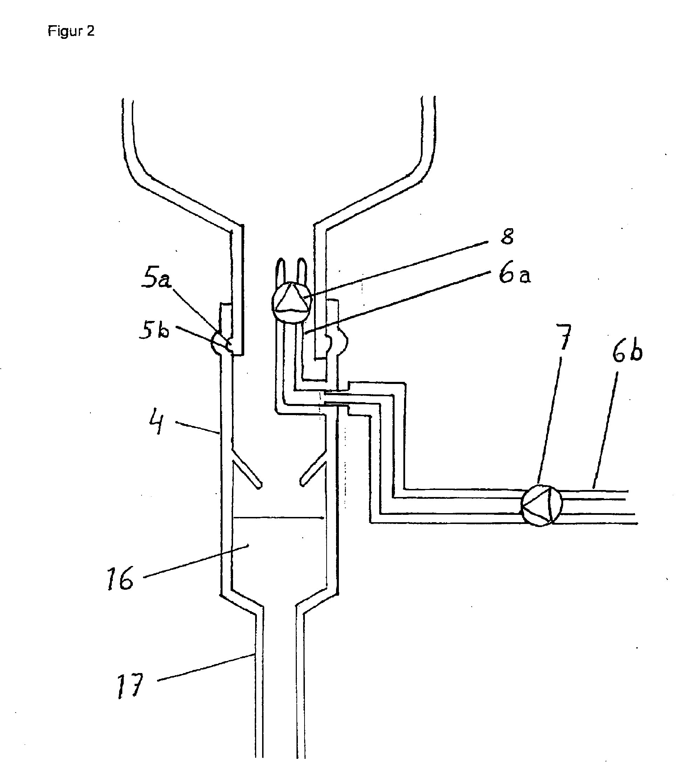

Figur 2 zeigt eine Variante der Verbindung zwischen dem Rohr 6a, welches in den Vorratsbehälter hineinragt, und dem Teil 6b als Ausschnittvergößerung. Bei dieser Variante sind Rohr 6a, Tropfkammer 16 und die Zufuhrleitung 17 zum Patienten einteilig ausgebildet. Die Verbindung zwischen dem Rohr 6a und dem Rohr 6b erfolgt durch eine Steckverbindung. Hierzu ist das mit dem Rohr 6b zu verbindende Ende des Rohres 6a rechteckig, vorzugsweise quadratisch, als Öffnung in dem Anschlussteil 4 ausgebildet. Das korrespondierende Ende des Rohres 6b ist in der Form an die Form der von dem Endes des Rohres 6a gebildeten Öffnung angepasst. In dieser Öffnung wird das Rohr 6b, welches zumindest an diesem Ende als elastischer Schlauch ausgebildet ist, dichtend aufgenommen. Alternativ kann auch ein separates Verbindungsstück in die Öffnung des Rohrteiles 6a und das Ende des Rohrteiles 6b eingesteckt werden. Durch den eckigen Querschnitt der Öffnung wird ein Verdrehen verhindert, bei dem eine erhebliche Gefahr bestünde, dass die Verbindung unabsichtlich gelöst wird. Ein weiterer Vorteil dieser Gestaltung liegt darin, dass sich ein nur in einem Punkt modifiziertes herkömmliches Überleitsystem einsetzen lässt, welches mittels Spritzguss preiswert gefertigt werden kann.

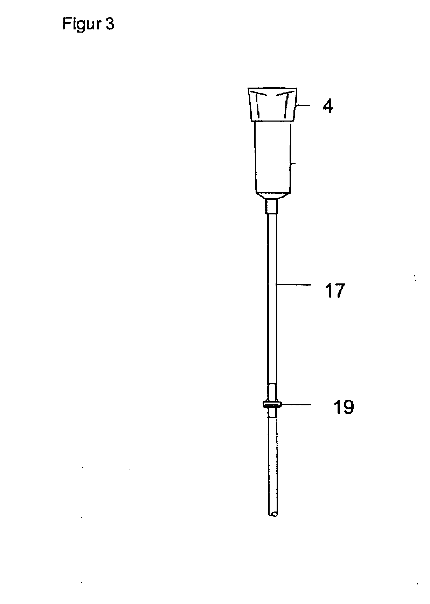

Die Figur 3 veranschaulicht eine bevorzugte Ausführungsform des Überleitsystems, bei dem in die Zuführungsleitung 17 ein Membranventil 19 eingebaut ist, welches ein Zurückströmen der Nahrung in Richtung auf den Vorratsbehälter 1 (nicht gezeigt) ausschließt.

### Bezugszeichenliste

- 1: Vorratsbehälter
- 2: Suspension
- 3: Luftraum
- 4: Anschlussteil
- 5: Verbindung zwischen Anschlussteil und Behälter
- 5a: Wulst am Behälter
- 5b: Nut am Anschlussteil
- 6: Rohr
- 6a: Teil des Rohres, der in den Behälter ragt
- 6b: Teil des Rohres mit der Lufteinlassöffnung
- 7: Einwegventil
- 8: Rückschlagventil
- 9: Nadelventil
- 10: Skalierung
- 11: Getriebe
- 12: Lufteinlassöffnung
- 13: bakteriendichter Filter
- 14: Absperrventil
- 15: Gehäuse
- 16: Tropfkammer
- 17: Zuführungsleitung
- 18: Schlaucholive an dem Anschlussteil
- 19: Membranventil

## Patentansprüche

1. Dosiervorrichtung für Flüssigkeiten, umfassend eine Anschlussvorrichtung zur Verbindung eines Vorratsbehälters, in dem eine Flüssigkeit enthalten ist, mit einem Empfänger, wobei die Anschlussvorrichtung einen Anschluss zum Verbinden mit dem Vorratsbehälter, eine Tropfkammer und eine Zuführungsleitung zum Empfänger umfasst, sowie ein Rohr, dessen einer Teil in den Vorratsbehälter mit der Flüssigkeit hineinragt und dessen anderer Teil mit einer Lufteinlassöffnung verbunden ist, **dadurch gekennzeichnet, dass** das Rohr (6) über ein Ventil (9) mit der Lufteinlassöffnung (12) verbunden ist, wobei das Ventil (9) eine Skalierung (10) aufweist und eine präzise und reproduzierbare Dosierung ermöglicht.

2. Dosiervorrichtung gemäß Anspruch 1, **dadurch gekennzeichnet, dass** die Flüssigkeit eine Lösung oder Suspension für die enterale Ernährung und der Empfänger ein Patient ist.

3. Dosiervorrichtung gemäß Anspruch 1 oder 2, **dadurch gekennzeichnet, dass** das Ventil (9) ein Nadelventil ist.

4. Dosiervorrichtung gemäß Anspruch 3, **dadurch gekennzeichnet, dass** das Nadelventil (9) mit einem Getriebe (11) zur Spreizung des Regelbereichs versehen ist.

5. Dosiervorrichtung gemäß mindestens einem der Ansprüche 1 bis 4, **dadurch gekennzeichnet, dass** das Ventil (9) elektronisch gesteuert ist.

6. Dosiervorrichtung gemäß mindestens einem der Ansprüche 1 bis 5, **dadurch gekennzeichnet, dass** das Rohr (6) in einen ersten Teil (6a), der durch das Anschlussteil (4) hindurch in den Behälter (1) hineinragt und einen zweiten Teil (6b), in welchem das Ventil (9) und die Lufteinlassöffnung (12) angeordnet sind, unterteilt wird und die beiden Teile (6a und 6b) miteinander verbindbar sind.

7. Dosiervorrichtung gemäß Anspruch 6, **dadurch gekennzeichnet, dass** die beiden Teile (6a und 6b) mit einer Schraubverbindung verbindbar sind.

8. Dosiervorrichtung gemäß Anspruch 6 oder 7 **dadurch gekennzeichnet, dass** ein Einwegventil (7) den zweiten Teil (6b) vor einem Rück- bzw. Einströmen von Luft schützt.

9. Dosiervorrichtung gemäß mindestens einem der Ansprüche 1 bis 8 **dadurch gekennzeichnet, dass** die Lufteinlassöffnung (12) mit einem bakteriendichten Filter (13) versehen ist.

10. Dosiervorrichtung gemäß mindestens einem der Ansprüche 1 bis 9 **dadurch gekennzeichnet, dass** der Teil des Rohres (6), der in den Vorratsbehälter (1) hineinragt, mit einem Rückschlagventil (8) versehen ist.

11. Dosiervorrichtung gemäß mindestens einem der Ansprüche 1 bis 10 **dadurch gekennzeichnet, dass** in dem Rohr (6) ein Absperrventil (14), mit welchem die Luftzufuhr unterbrochen werden kann, als An- und Abschaltschalter vorgesehen ist.

12. Dosiervorrichtung gemäß mindestens einem der Ansprüche 1 bis 11 **dadurch gekennzeichnet, dass** der Teil des Rohres (6), in dem das Ventil (9) und die Lufteinlassöffnung (12) angeordnet sind, in einem Gehäuse (15) eingeschlossen ist, wobei sich ein Teil des Ventils (9), mit welchem dieses betätigt wird, außerhalb des Gehäuses (15) befindet.

13. Dosiervorrichtung gemäß Anspruch 6, **dadurch gekennzeichnet, dass** die beiden Teile (6a und 6b) mit einer Steckverbindung verbindbar sind.

14. Dosiervorrichtung gemäß Anspruch 13, **dadurch gekennzeichnet, dass** die Steckverbindung von einem rechteckigen Ende aus elastischem Material des einen Teiles (6b) und einer in der Form entsprechenden Öffnung in einem Ende des anderen Teiles (6a), in die das rechteckige Ende dichtend aufnehmbar ist, gebildet wird.

15. Dosiervorrichtung gemäß einem der Ansprüche 1 bis 14, **dadurch gekennzeichnet, dass** in der Zuführungsleitung (17) zum Empfänger oder in der Tropfkammer (16) ein Einwegeventil (19), vorzugsweise ein Membranventil, zur Verhinderung eines Rückströmens der Flüssigkeit in Richtung auf den Vorratsbehälter (1) vorgesehen ist.
